# EUROPÄISCHE PATENTSCHRIFT

(11) **EP 2 099 520 B1**
(45) Veröffentlichungstag und Bekanntmachung des Hinweises auf die Patenterteilung: **02.06.2010**
(21) Anmeldenummer: 07856443.2
(22) Anmeldetag: 07.12.2007
(51) Int. Cl.: A61N 1/39

(54) **DEFIBRILLATOR**
DEFIBRILLATOR
DÉFIBRILLATEUR

(30) Priorität: 07.12.2006 DE 202006018672 U
(43) Veröffentlichungstag der Anmeldung: 16.09.2009
(73) Patentinhaber: Metrax GmbH, 78628 Rottweil (DE)
(72) Erfinder: ROSO, Bruno, 78073 Bad Dürrheim (DE); V. WAGNER, Gero, 78628 Rottweil (DE)
(74) Vertreter: Fleck, Hermann-Josef
(86) Internationale Anmeldenummer: PCT/EP2007/010638
(87) Internationale Veröffentlichungsnummer: WO 2008/068028

(56) Entgegenhaltungen:
- WO-A-01/26732
- WO-A-03/009895
- DE-A1- 10 028 410
- US-A1- 2003 004 547

## Beschreibung

Die Erfindung bezieht sich auf einen Defibrillator für ein äußeres Anlegen an einem Patienten mit einer Energiespeichereinheit zur Abgabe eines Defibrillationsschocks, die eine Kondensatoreinheit mit mindestens einem Kondensator aufweist.

Ein Defibrillator dieser Art ist in der DE 100 15 152 A1 gezeigt. Eine in den Defibrillator eingebaute Energiespeichereinheit für einen an einem Patienten anzulegenden Defibrillationsimpuls weist mehrere Kondensatoren auf, die in das Gerät integriert sind und in unterschiedlicher Weise über gesteuerte Schalter in Serien- bzw. Parallelschaltung zur Bereitstellung der Defibrillationsenergie miteinander elektrisch verbunden werden können. Auf diese Weise ist eine Anpassung an unterschiedliche Impedanzen von Patienten möglich. Dieser Aufbau bedingt eine relativ aufwändige Steuerungstechnik.

Einen ähnlichen Aufbau zeigt auch ein in der US 2003/0004547 A1 angegebener Defibrillator, wobei für die Entladung fünf Kondensatoren in verschiedenen Konfigurationen in Serie und parallel geschaltet werden können.

Die Adaption des Defibrillationsschocks an die Patientenimpedanz ist in verschiedenen Druckschriften gezeigt, z. B. in US 6,493,580 mit Anpassung der Impulsdauer an die Patientenimpedanz und mit getaktetem BTE-Impuls (Biphasic Truncated Exponential), in US 5,372,606 mit Variation der Impulslänge abhängig von der Patientenimpedanz und in DE 102 54 481 B3 mit stromgesteuerter Endstufe.

Werden mehrere Kondensatoren als Energiespeicher verwendet, dient dies in den meisten Fällen dazu, die Energiebereitstellung durch Parallelschaltung der Kondensatoren zu erleichtern. Für die Schockabgabe werden sie dann in Reihe geschaltet.

In der DE 197 51 024 A1 ist ein Defibrillator mit fest eingebauter Kondensatoreinheit und ankoppelbaren Zusatzgeräten gezeigt, die aber nicht die eigentliche Defibrillatorelektronik des Grundgeräts betreffen. Entsprechendes gilt auch für einen in der DE 100 28 410 A1 dargestellten Defibrillator.

Dokument WO 01/26732 betrifft ein Defibrillator gemäss dem Oberbegriff des Anspruchs 1.

Der Erfindung liegt die Aufgabe zugrunde, einen den Eigenschaften eines Patienten besser angepassten äußeren Defibrillator bereit zu stellen.

Diese Aufgabe wird mit den Merkmalen des Anspruches 1 gelöst. Hierbei ist vorgesehen, dass mehrere unterschiedliche Kondensatoreinheiten mit auf verschiedene

Patientenimpedanzen abgestimmter Kapazität vorgesehen sind, die an dem Defibrillator austauschbar angekoppelt oder ankoppelbar sind. Hierdurch ergibt sich eine Anpassung der Kapazität der Energiespeichereinheit in Form einer Kondensatoreinheit eines externen Defibrillators an die individuelle Impedanz eines Patienten zum Erreichen bestimmter Kenngrößen wie Stromstärke und -verlauf während eines Defibrillationsschocks. Die Kondensatoreinheiten sind als aus dem Defibrillatorsystem entnehmbare Module ausgestaltet und leicht entnehmbar und einsetzbar.

Eine einfache, sichere Handhabung und leichte Bedienung, ergeben sich dadurch, dass die Kondensatoreinheiten in jeweiligen Aufnahmemodulen aufgenommen und mit diesen austauschbar an dem Defibrillator angekoppelt oder ankoppelbar sind.

Eine einfache, eindeutige Zuordnung eines Aufnahmemoduls in Abhängigkeit von einem bestimmten Patienten wird z. B. bei einem "klassischen" Defibrillator im Notfalleinsatz (Rettungsdienst, Behandlung durch einen Notarzt) dadurch gewährleistet, dass der Defibrillator mit einer Messvorrichtung zum Erfassen der Patientenimpedanz ausgestattet ist und dass eine Informationsausgabe vorhanden ist, über die eine auf die Impedanz abgestimmte Kondensatoreinheit dem Defibrillator zuordenbar ist. Hierbei bestehen vorteilhafte Ausgestaltungsvarianten darin, dass die Information akustisch und/oder optisch ausgebbar ist und direkt oder indirekt auf das anzukoppelnde Aufnahmemodul bezogen ist.

Weitere vorteilhafte Maßnahmen für eine eindeutige Zuordnung einer geeigneten Kondensatoreinheit in Abhängigkeit von der Impedanz eines zu behandelnden Patienten bestehen darin, dass die unterschiedlichen Aufnahmemodule mit unterschiedlichen, bezüglich der jeweiligen Kapazität eindeutigen Kennzeichnungen versehen sind. Beispielsweise können die Aufnahmemodule einer bestimmten Kapazität alle mit einer roten Markierung oder einem anderen eindeutigen, schnell zu erfassenden Hinweis gekennzeichnet sein, während alle Aufnahmemodule mit einer anderen Kapazität für eine andere Patientenimpedanz z. B. grün oder mit einem anderen eindeutigen Hinweis versehen sind. Die Grenzen der Impedanzbereiche, denen jeweilige Kondensatoreinheiten zugeordnet sind, können geeignet festgelegt werden, und auch später können, soweit sich entsprechende Erkenntnisse ergeben und dies zweckmäßig ist, andere Impedanzbereiche und andere diesen zugeordnete Kondensatoreinheiten gewählt bzw. bereitgestellt werden. Es sollten mindestens zwei, besser drei oder mehr Kondensatoreinheiten verschiedener Kapazität vorhanden sein.

Einem weiteren wichtigen Anwendungsbereich wird dadurch Rechnung getragen, dass der Defibrillator als dauerhaft von einem Patienten zu tragendes Gerät ausgebildet und die Messvorrichtung zur dauerhaften Überwachung der Patientenimpedanz und mit einer Auswertevorrichtung ausgestaltet ist und dass die Auswertevorrichtung zur Abgabe eines Warnsignals ausgebildet ist, wenn eine vorgegebene kritische Grenze bei Änderung der Patientenimpedanz über- oder unterschritten wird.

Eine vorteilhafte Abstimmung und Zuordnung wird dadurch erreicht, dass die Kapazität der Kondensatoreinheit in Abhängigkeit mindestens eines Parameterwerts des an den Patienten anzulegenden Defibrillationsimpulses bestimmt ist.

Vorteilhafte Ausgestaltungen bestehen dabei darin, dass der mindestens eine Parameterwert des Defibrillationsimpulses die Energie, das Amplitudenverhältnis des Stroms zu Beginn und am Ende einer lmpulsphase oder die Stromstärke zu Beginn einer Impulsphase oder eine Kombination mindestens zweier dieser Größen betrifft.

Zu einer sicheren Bedienung und zuverlässigen Funktion tragen die Maßnahmen bei, dass zum Absichern der Kondensatoreinheit, insbesondere bei vorhandener Restspannung ein automatisch wirkender Abdeckungsmechanismus vorgesehen ist, der so ausgebildet ist, dass er beim Ankoppeln des Aufnahmemoduls automatisch entriegelt und bei der Entnahme automatisch verriegelt wird.

Vorteilhafte Maßnahmen bestehen dabei darin, dass der Abdeckungsmechanismus mit einem Entladewiderstand versehen ist, über den auf eine Entnahme des Aufnahmemoduls hin eine kontrollierte Entladung der Kondensatoreinheit erfolgt.

Mit diesen Maßnahmen wird ein versehentlicher Kurzschluss des Kondensators bei vorhandener Restspannung vermieden.

Der Erfindung liegen folgende Überlegungen und Erkenntnisse zugrunde: Normalerweise besitzen Defibrillatoren eine Kondensatoranordnung fester Größe, dessen Kapazität so gewählt ist, dass über einen möglichst großen Bereich von Patientenimpedanzen in Kombination mit angepassten Hochspannungen Ströme in einer physiologisch wirksamen Form (Amplitude und Zeitverlauf betreffend) durch den Körper getrieben werden. Eine verbreitete Impulsform ist der so genannte Biphasic-Truncated-Exponential (BTE)-Puls, bei dem der auf Hochspannung geladene Kondensator über den Patientenwiderstand durch entsprechende Steuerung biphasisch entladen wird.

Es sind besonders folgende Probleme zu sehen:
- Hohe Spitzenströme bei niedriger Patientenimpedanz mit Gefahr der Gewebeschädigung;
- niedrige Patientenströme bei hohen Impedanzen, die den Defibrillationserfolg gefährden können;
- starker Stromabfall (kleines Verhältnis der Stromamplituden am Ende und am Anfang der Schockphase) bei niedrigen Impedanzen, die den Defibrillationserfolg gefährden können.

Diese Nachteile werden durch die erfindungsgemäßen Maßnahmen vermieden.

Für ein dauerhaft zu tragendes Monitoringsystem mit integrierter Defibrillationseinheit ist die patientenindividuelle Impedanz im Voraus bekannt oder wird mittels einer Messvorrichtung erfasst. Durch körperliche Anstrengung, zirkadiane Variation etc. kann es auch hier zu Schwankungen der Patientenimpedanz kommen, jedoch in der Regel nicht in der gesamten Bandbreite. Erfindungsgemäß wird nun für ein Defibrillationssystem ein in der (geometrischen) Größe standardisiertes Kondensatoraufnahmemodul geschaffen, das einen Kondensatorsatz (Kondensatoreinheit) mit auf den individuellen Patienten abgestimmter Kapazität enthält. Die Abstufung der abgedeckten Patientenimpedanzen kann mehr oder weniger grob ausgelegt werden.

Die Erfindung wird nachfolgend anhand von Ausführungsbeispielen unter bezugnahme auf die Zeichnungen näher erläutert. Es zeigen:
- Fig. 1a: ein Kondensatoraufnahmemodul in schematischer perspektivischer An- sicht,
- Fig. 1b: einen Defibrillator mit einer in einem Kondensatoraufnahmemodul nach Fig. 1a aufgenommene Kondensatoreinheit in schematischer Darstel- lung,
- Fig. 2: einen Ausschnitt eines Kondensatoraufnahmemoduls nach Fig. 1a bei freigegebenen Anschlusskontakten und vor Einsatz in einen Einschub- schacht in einem Defibrillatorgehäuse,
- Fig. 3: eine Schnittdarstellung einer in Fig. 2 gezeigten Schutzkappe in einer Verschlussstellung mit darin angeordneten Komponenten und
- Fig. 4a, 4b und 4c: eine Kondensatoranordnung der Kondensatoreinheit vor einer Aufla- dung, bei einer Aufladung auf Hochspannung in Parallelschaltung bzw. bei Entladung.

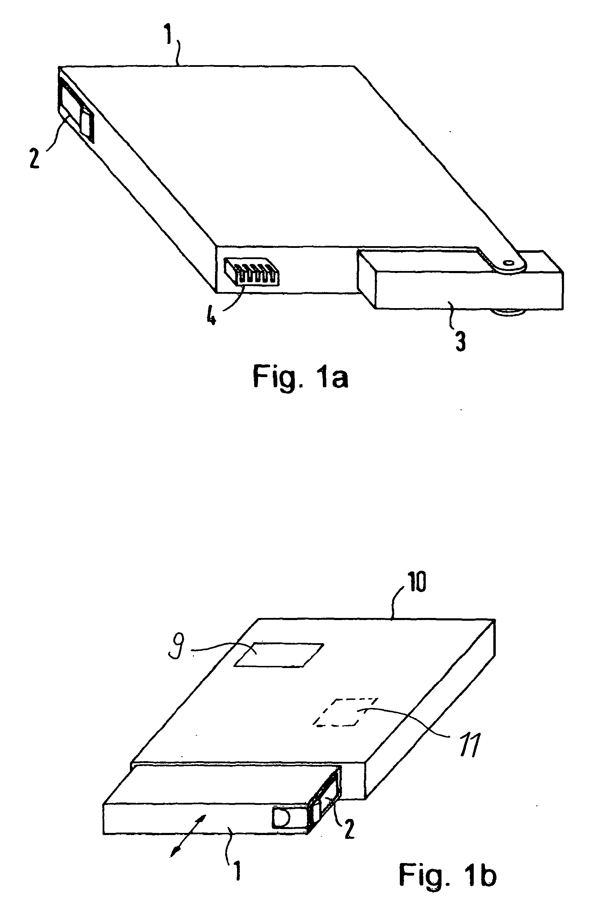

Fig. 1a und 1b zeigen ein Beispiel für den Aufbau eines Kondensatoraufnahmemoduls 1. Das Kondensatoraufnahmemodul kann in das Defibrillationssystem eingeschoben und verriegelt werden, wie Fig. 1b zeigt. Durch eine Entriegelung auf der Bodenseite kann das Modul wieder entnommen werden.

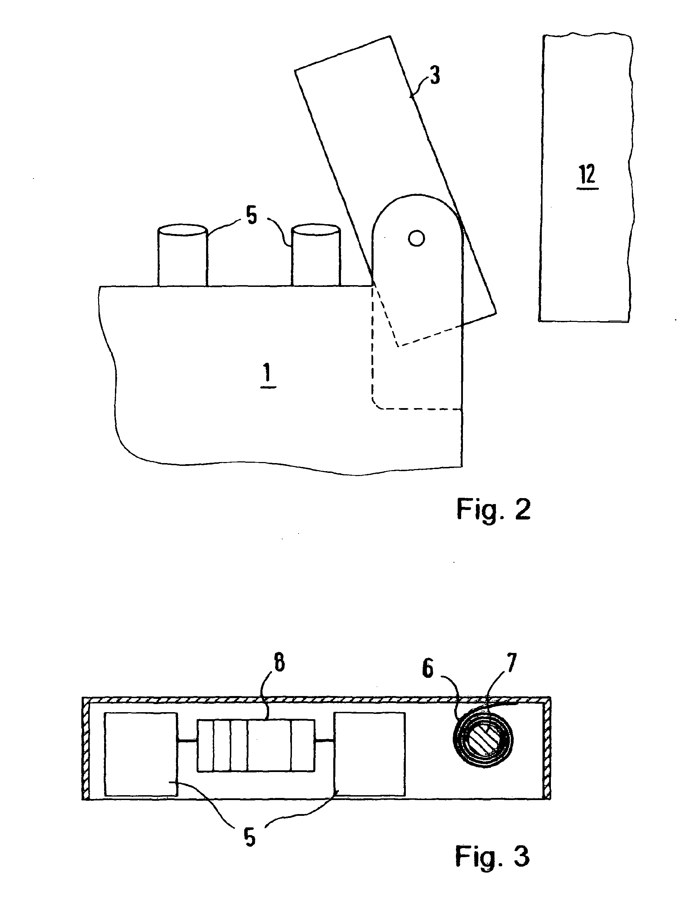

Fig. 2 und 3 zeigen ein mögliches Ausführungsbeispiel eines Schutzmechanismus. Das Kondensatoraufnahmemodul 1 enthält für den sicheren Abbau von etwaig vorhandener Restspannung einen Mechanismus mit einer Vorrichtung, die bei Entnahme des Kondensatormoduls aus dem Defibrillatorsystem für eine sichere Entladung über einen Lastwiderstand (Entladewiderstand 8, s. Fig. 3) sorgt. Wie Fig. 2 zeigt, weist dieser Mechanismus eine Schutzkappe 3 mit dem integrierten Entladewiderstand 8 auf, die beim Einschieben des Kondensatoraufnahmemoduls 1 in das Defibrillationssystem 10 mechanisch selbsttätig um ein Scharnier 7 aufgeklappt wird und die darunterliegenden Anschlusskontakte 5 der Kondensatoranodnung freigibt. Beim Entfernen des Aufnahmemoduls 1 aus dem Defibrillationssystem schnappt die Schutzkappe 3 um das Scharnier 7 durch Federkraft (Feder 6) in ihre Schließposition. Auch andere Ausführungsformen von Aufnahmemodulen sind möglich, z. B. eine lediglich ansteckbare oder einschwenkbare Ausgestaltung.

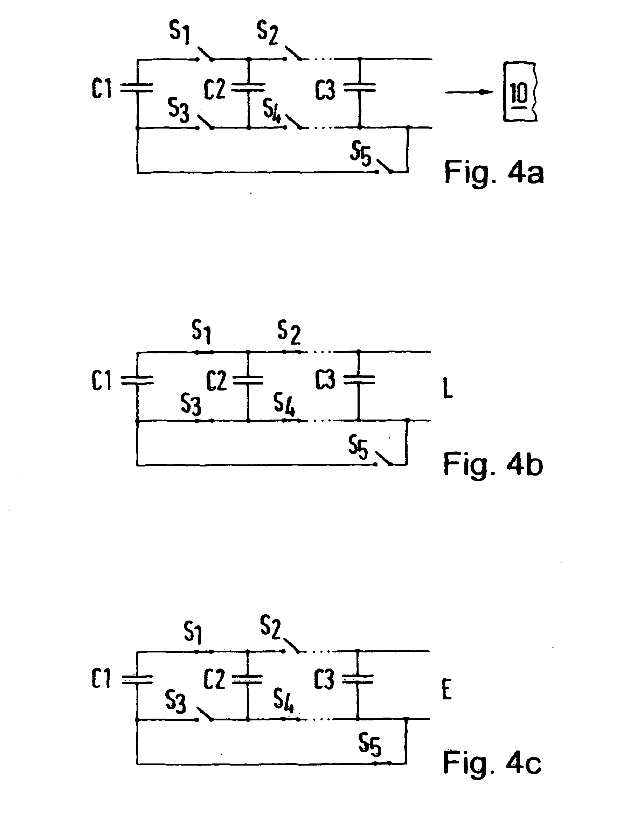

Wie Fig. 4 zeigt, kann das Einschubmodul 1 zusätzlich aktive Elemente S1, S2, S3, S4, S5 enthalten, die über eine Steuereinrichtung und zusätzliche Steuerleitungen eine Parallelschaltung von mehreren Kondensatoren C1, C2, C3 im Modul 1 während der Aufladung L (mittlere Darstellung) und deren Serienschaltung für die Entladung E (untere Darstellung) ermöglicht.

Auf Basis der genannten Maßnahmen wird ein individuell auf einen Patienten abgestimmtes Defibrillatorsystem 10 für den dauerhaften Gebrauch bereitgestellt, bei dem die für die Defibrillation notwendige Energie in einer Kondensatoranordnung C1, C2, C3 gespeichert wird, deren Kapazität auf die jeweilige Patientenimpedanz abgestimmt ist. Das Defibrillationssystem 10 kann aus mehreren Teilmodulen aufgebaut sein.

Die Kapazität wird vorteilhaft durch folgende Parameter der impulsform bestimmt: Energie und Amplitudenverhältnis des Stroms zu Beginn und am Ende einer Impulsphase. Zusätzlich oder alternativ ist vorgesehen, dass die Kapazität durch folgende Parameter der Impulsform bestimmt wird: Stromstärke zu Beginn einer Impulsphase und Amplitudenverhältnis des Stroms zu Beginn und Ende einer Impulsphase.

Die Kondensatoranordnung mit der patientenadaptierten Kapazität ist als Einheit in einem standardisierten Modul untergebracht, das in das Defibrillationssystem 10 oder eines seiner Teilmodule eingeschoben werden kann.

Das Einschubmodul 1 enthält mindestens einen Kondensator C1, C2, C3 und eine Vorrichtung, die sicherstellt, dass bei Entnahme des Einschubmoduls 1 aus dem Defibrillationssystem evtl. vorhandene Restspannung über die in das Einschubmodul 1 integrierte Last- bzw. den Entladewiderstand 8 vernichtet wird. Über separate Steuerleitungen wird sicherstellt, dass mehrere in dem Einschubmodul 1 integrierte Kondensatoren C1, C2, C3 parallel und in Serie geschaltet werden können.

Wie Fig. 1b zeigt, ist der Defibrillator vorteilhaft mit einer Messvorrichtung 11 zur Messung einer Patientenimpedanz sowie einer Anzeigevorrichtung 9 ausgestattet, über die eine mit der Patientenimpedanz zusammenhängende Anzeige z. B. akustisch und/oder optisch geboten wird, so dass eine Bedienperson eine Information darüber erhält, welche Kondensatoreinheit bzw. welches Kondensatoraufnahmemodul 1 für die Behandlung des Patienten mit dem Defibrillator am geeignetsten ist. Auf diese Weise erhält z. B. bei einem "klassischen" Defibrillator im Notfalleinsatz die Rettungsperson bzw. der Notarzt schnell einen Hinweis darauf, welches Aufnahmemodul 1 er zur Behandlung des betreffenden Patienten einsetzen soll. Vorteilhaft sind die Anzeige und eine Kennzeichnung des Aufnahmemoduls 1 bereits aufeinander abgestimmt, z. B. "rotes Modul", "grünes Modul" ..., so dass der Einsatz des Kondensatoraufnahmemoduls in dem Defibrillatorgehäuse besonders einfach ist.

Bei einem Defibrillator, der für ein dauerhaftes Tragen an einem Patienten ausgebildet ist, kann die Messvorrichtung 11 des Weiteren mit einer Auswertevorrichtung versehen sein, die die Patientenimpedanz dauernd überwacht und zudem feststellt, wenn diese eine vorgegebene kritische Grenze überschreitet bzw. unterschreitet und ein Wechsel zweckmäßig ist. In diesem Fall besteht eine weitere vorteilhafte Ausgestaltung darin, dass über die Anzeigevorrichtung 9 dem Anwender bzw. Patienten ein Warnhinweis übermittelt wird, dass Anpassungsbedarf besteht und zum Wechsel des Aufnahmemoduls aufgefordert wird.

## Patentansprüche

1. Defibrillator für ein äußeres Anlegen an einem Patienten mit einer Energiespeichereinheit zur Abgabe eines Defibrillationsschocks, die eine Kondensatoreinheit mit mindestens einem Kondensator (C1, C2, C3) aufweist, wobei die Kondensatoreinheit eine von mehreren Kondensatoreinheiten ist, die an dem Defibrillator angekoppelt oder ankoppelbar sind **dadurch gekennzeichnet,**
**dass** die Kondensatoreinheiten austauschbar sind, und unterschiedliche Kondensatoreinheiten mit auf verschiedene Patientenimpedanzen abgestimmter Kapazität sind.

2. Defibrillator nach Anspruch 1,
**dadurch gekennzeichnet,**
**dass** die Kondensatoreinheiten in jeweiligen Aufnahmemodulen (1) aufgenommen und mit diesen austauschbar an dem Defibrillator angekoppelt oder ankoppelbar sind.

3. Defibrillator nach Anspruch 1 oder 2,
**dadurch gekennzeichnet,**
**dass** der Defibrillator eine Messvorrichtung (11) zum Erfassen der Patientenimpedanz ausgestattet ist und dass eine Informationsausgabe (9) vorhanden ist, über die eine auf die Impedanz abgestimmte Kondensatoreinheit dem Defibrillator zuordenbar ist.

4. Defibrillator nach Anspruch 3,
**dadurch gekennzeichnet,**
**dass** die Information akustisch und/oder optisch ausgebbar ist und auf das anzukoppelnde Aufnahmemodul (1) bezogen ist.

5. Defibrillator nach einem der vorhergehenden Ansprüche,
**dadurch gekennzeichnet,**
**dass** die unterschiedlichen Aufnahmemodule (1) mit unterschiedlichen, bezüglich der jeweiligen Kapazität eindeutigen Kennzeichnungen versehen sind.

6. Defibrillator nach einem der Ansprüche 3 bis 5,
**dadurch gekennzeichnet,**
**dass** der Defibrillator als dauerhaft von einem Patienten zu tragendes Gerät ausgebildet und die Messvorrichtung zur dauerhaften Überwachung der Patientenimpedanz mit einer Auswertevorrichtung ausgestaltet ist und dass die Auswertevorrichtung zur Abgabe eines Warnsignals ausgebildet ist, wenn eine vorgegebene Grenze bei Änderung der Patientenimpedanz über-oder unterschritten wird.

7. Defibrillator nach einem der vorhergehenden Ansprüche,
**dadurch gekennzeichnet,**
**dass** die Kapazität der Kondensatoreinheit in Abhängigkeit mindestens einem Parameterwert des bei dem an den Patienten anzulegenden Defibrillationsimpulses bestimmt ist.

8. Defibrillator nach Anspruch 7,
**dadurch gekennzeichnet,**
**dass** der mindestens eine Parameterwert des Defibrillationsimpulses die Energie, das Amplitudenverhältnis des Stroms zu Beginn und am Ende einer Impulsphase oder die Stromstärke zu Beginn einer Impulsphase oder eine Kombination mindestens zweier dieser Größen betrifft.

9. Defibrillator nach einem der vorhergehenden Ansprüche,
**dadurch gekennzeichnet,**
**dass** zum Absichern der Kondensatoreinheit bei vorhandener Restspannung ein automatisch wirkender Abdeckungsmechanismus vorgesehen ist, der so ausgebildet ist, dass er beim Ankoppeln des Aufnahmemoduls (1) automatisch entriegelt und bei der Entnahme automatisch verriegelt wird.

10. Defibrillator nach Anspruch 9,
**dadurch gekennzeichnet,**
**dass** der Abdeckungsmechanismus mit einem Entladewiderstand (8) versehen ist, über den auf eine Entnahme des Aufnahmemoduls (1) hin eine kontrollierte Entladung der Kondensatoreinheit (C1, C2, C3) erfolgt.

## Claims

1. Defibrillator for external application to a patient, with a power storage unit for supplying a defibrillation shock, which has a capacitor unit with at least one capacitor (C1, C2, C3), the capacitor unit being one of several capacitor units which are or can be coupled to the defibrillator,
**characterised in**
**that** the capacitor units are replaceable, and different capacitor units have a capacity adapted to various patient impedances.

2. Defibrillator according to claim 1,
**characterised in**
**that** the capacitor units are housed in respective receiving modules (1) and are or can be coupled in a replaceable manner to the defibrillator therewith.

3. Defibrillator according to claim 1 or 2,
**characterised in**
**that** the defibrillator is equipped with a measurement device (11) for recording the patient's impedance and that a data output device (9) is present via which a capacitor unit adapted to the impedance can be assigned to the defibrillator.

4. Defibrillator according to claim 3,
**characterised in**
**that** the data can be output acoustically and/or optically and relates to the receiving module (1) to be coupled.

5. Defibrillator according to one of the preceding claims,
**characterised in**
**that** the different receiving modules (1) are provided with various distinctive markings relating to the respective capacity.

6. Defibrillator according to one of claims 3 to 5,
**characterised in**
**that** the defibrillator is designed as a device to be worn by a patient over a long period and the measurement device is equipped with an evaluation device to monitor the patient's impedance over a long period and that the evaluation device is designed to deliver a warning signal if there is any change in the patient's impedance to above or below a predetermined limit.

7. Defibrillator according to one of the preceding claims,
**characterised in**
**that** the capacity of the capacitor unit is determined as a function of at least one parameter value of the defibrillation pulse to be applied to the patient.

8. Defibrillator according to claim 7,
**characterised in**
**that** the at least one parameter value of the defibrillation pulse relates to the energy, the amplitude ratio of the current at a beginning and an end of a pulse phase, or the amperage at the beginning of a pulse phase, or a combination of at least two of these values.

9. Defibrillator according to the preceding claim,
**characterised in**
**that** in order to safeguard the capacitor unit if a residual voltage is present, an automatically acting covering mechanism is provided which is designed so that it automatically unlocks when the receiving module (1) is coupled into position and automatically locks upon removal.

10. Defibrillator according to claim 9,
**characterised in**
**that** the covering mechanism is provided with a discharging resistor (8) by means of which a controlled discharge of the capacitor unit (C1, C2, C3) occurs upon removal of the receiving module (1).

## Revendications

1. Défibrillateur pour une application extérieure sur un patient, comprenant une unité de stockage d'énergie servant à délivrer un choc de défibrillation, qui présente une unité de condensateurs contenant au moins un condensateur (C1, C2, C3), l'unité de condensateurs étant une de plusieurs unités de condensateurs qui sont couplées ou peuvent être couplées au défibrillateur,
**caractérisé en ce**
**que** les unités de condensateurs sont échangeables et que différentes unités de condensateurs ont des capacités adaptées à différentes impédances de patients.

2. Défibrillateur selon la revendication 1,
**caractérisé en ce**
**que** les unités de condensateurs sont logées dans des modules de logement (1) respectifs et sont couplées ou peuvent être couplées au moyen de ceux-ci de manière échangeable au défibrillateur.

3. Défibrillateur selon la revendication 1 ou 2,
**caractérisé en ce**
**que** le défibrillateur est équipé d'un dispositif de mesure (11) pour saisir l'impédance du patient et qu'il est prévu une sortie d'information (9) au moyen de laquelle une unité de condensateurs adaptée à l'impédance peut être associée au défibrillateur.

4. Défibrillateur selon la revendication 3,
**caractérisé en ce**
**que** l'information peut être émise par voie acoustique et/ou optique et se rapporte au module de logement (1) à coupler.

5. Défibrillateur selon une des revendications précédentes,
**caractérisé en ce**
**que** les différents modules de logement (1) sont munis d'identifications différentes, univoques en ce qui concerne leur capacité respective.

6. Défibrillateur selon une des revendications 3 à 5,
**caractérisé en ce**
**que** le défibrillateur est conçu comme appareil à porter en permanence par un patient et le dispositif de mesure équipé d'un dispositif d'évaluation pour la surveillance permanente de l'impédance du patient et
**que** le dispositif d'évaluation est conçu pour délivrer un signal d'avertissement quand une limite prédéfinie est dépassée vers le haut ou vers le bas en cas de changement de l'impédance du patient.

7. Défibrillateur selon une des revendications précédentes,
**caractérisé en ce**
**que** la capacité de l'unité de condensateurs est déterminée en fonction d'au moins une valeur de paramètre de l'impulsion de défibrillation à appliquer au patient.

8. Défibrillateur selon la revendication 7,
**caractérisé en ce**
**que** ladite au moins une valeur de paramètre de l'impulsion de défibrillation concerne l'énergie, le rapport d'amplitude du courant au début et à la fin d'une phase d'impulsion ou l'intensité de courant au début d'une phase d'impulsion ou une combinaison d'au moins deux de ces grandeurs.

9. Défibrillateur selon une des revendications précédentes,
**caractérisé en ce**
**que** pour assurer la sécurité de l'unité de condensateurs en cas de présence d'une tension résiduelle, il est prévu un mécanisme de recouvrement à fonctionnement automatique qui est conçu pour se déverrouiller automatiquement lors du couplage du module de logement (1) et pour se verrouiller automatiquement lors de son enlèvement.

10. Défibrillateur selon la revendication 9,
**caractérisé en ce**
**que** le mécanisme de recouvrement est muni d'une résistance de décharge (8) via laquelle, suite à l'enlèvement du module de logement (1), une décharge contrôlée de l'unité de condensateurs (C1, C2, C3) a lieu.
